# EUROPÄISCHE PATENTSCHRIFT

(11) **EP 0 956 900 B1**
(45) Veröffentlichungstag und Bekanntmachung des Hinweises auf die Patenterteilung: **13.10.2004**
(21) Anmeldenummer: 99109038.2
(22) Anmeldetag: 07.05.1999
(51) Int. Cl.: B01L 11/00, G01N 1/10, A61B 5/15

(54) **Vorrichtung zur Entnahme einer Flüssigkeitsprobe aus einem Schlauchstück**
Device for taking liquid samples from flexible tubing
Dispositif de prélèvement d'échantillons à partir de liquides contenus dans des tuyaux souples

(30) Priorität: 11.05.1998 DE 29808474 U
(43) Veröffentlichungstag der Anmeldung: 17.11.1999
(73) Patentinhaber: Eltest -Gesellschaft f. Elektrophorese- und Transfusionssysteme mbH, 53111 Bonn (DE)
(72) Erfinder: Muschiol, Norbert, 53505 Berg (DE)
(74) Vertreter: Valentin, Ekkehard

(56) Entgegenhaltungen:
- DE-C- 19 644 644
- FR-A- 2 759 780
- US-A- 5 714 125

## Beschreibung

Die Erfindung betrifft eine Vorrichtung zur Entnahme einer Flüssigkeitsprobe aus einem Schlauchstück, das eine verbreiterte Querschweißnaht aufweist, mit einem zylindrischen Körper mit einer Sacklochöffnung, einer an deren Umfangswand ausgebildeten, der Querschnittsform der Querschweißnaht des Schlauchstückes entsprechenden Ausbuchtung zum Einsetzen der Querschweißnaht des Schlauchstückes und einer in der Längsachse angeordneten Kanüle, die in einer konzentrisch zur Längsachse verlaufenden Durchgangsbohrung eines an den Körper angeformten Anschlußstückes gehalten ist.

Im allgemeinen müssen insbesondere Blutkonserven nach deren Entnahme vom Patienten oder vor der Infusion mehrmals getestet werden, beispielsweise um eine Blutgruppe festzustellen oder zu prüfen, ob die Blutkonserve mit AIDS-Erregern infiziert ist. Jeder Blutkonserve sind dabei Proben in Form von mehreren Begleitröhrchen mit mehreren Millilitern Volumen beigefügt. Derartige Proben haben jedoch den Nachteil, daß sie nur begrenzte Zeit gelagert werden können, wenn sie einmal geöffnet wurden. Darüber hinaus kann die Probe nach dem Öffnen verunreinigt werden, was zu falschen Meßergebnissen führen kann.

Es ist daher bekannt, die erforderlichen Blutproben in Schlauchsegmente einzuschließen, um den Anforderungen an Sicherheit und Hygiene gerecht zu werden. Zu diesem Zweck ist an jeden Blutbeutel ein extrudierter Kunststoffschlauch angebracht, der in bestimmten Abständen Querverschweißungen aufweist, die den Schlauch in hermetisch abgeschlossene Segmente unterteilen. Jeder dieser Schlauchsegmente umfaßt ein bestimmtes Volumen von Blut. Um die Blutkonserve zu testen, ist daher ein endseitig angeordneter Schlauchabschnitt des Schlauches abzuschneiden und zu öffnen. Hierfür wird in der Regel eine Schere verwendet, wobei es sowohl zum Verspritzen von Blut als auch zur Hämolyse kommen kann.

Aus der EP 0 499 764 A1 ist ein Anschlußstück aus im wesentlichen Kunststoff bekannt, das ein glockenförmiges Aufnahmeteil mit einer darin aufgenommenen Kanüle aufweist. Dieses wird auf eine eine medizinische Flüssigkeit enthaltene Flasche aufgesetzt, so daß die Kanüle in einen durchstoßbaren Verschluß der Flasche eindringen und die Flüssigkeit beispielsweise in einen am Anschlußstück befestigten Schlauch ableiten kann.

Durch die DE 295 18 814 U1 ist eine Vorrichtung zum Öffnen einer Blutprobe bekanntgeworden, bei der ein durch Querverscheißung begrenzter Schlauchabschnitt verwendet wird, der eine Blutprobe einschließt. Um die Blutentnahme zu Testzwecken zu ermöglichen, ist ein einseitig offener rohrförmiger Körper vorgesehen, in dem etwa parallel bzw. konzentrisch zur Längsmittelachse eine Kanüle angeordnet ist, deren Einstechspitze zur offenen Stirnseite des rohrförmigen Körpers weist und deren Kanal den Boden des rohrförmigen Körpers durchsetzt. Im Inneren des rohrförmigen Körpers sind Führungen für den Schlauchabschnitt angebraucht, die eine Kollision zwischen der Kanülenspitze und der Querschweißnaht des Schlauchabschnittes verhindern sollen.

Aus der DE 196 44 644 C1 ist eine Vorrichtung zur Entnahme einer Flüssigkeitsprobe aus einem Schlauchstück bekannt, bei der das Schlauchstück nicht lotrecht entlang der Längsmittelachse des zylindrischen Körpers, sondern in schräger Weise seitlich des zylindrischen Körpers eingeführt wird, indem der Schlauchabschnitt in eine zur Spitze der Kanüle fluchtende Ausbuchtung eingelegt wird, so daß die Querschweißnaht ins Innere des zylindrischen Körpers ragt. Dabei ist die Form der Ausbuchtung einem Teil des Innenumfanges des Schlauchstückes angepaßt, d.h., daß die Ausbuchtung in ihrer zur Kanülenspitze gerichteten Längsachse gewölbt ist. Da die Ausbuchtung im wesentlichen zur äußeren Spitze der Kanüle fluchtet, gleitet die Querschweißnaht am vorderen Ende des Schlauchstückes ohne Behinderung über die Kanülenspitze hinweg und stößt an die der Ausbuchtung gegenüberliegenden Innenwand des zylindrischen Körpers. Damit hintergreift die Querschweißnaht des Schlauchstückes gewissermaßen die Spitze der Kanüle, wodurch diese in jedem Fall hinter der Querschweißnaht in das die Flüssigkeitsprobe enthaltende Segment des Schlauchstückes einsticht. Eine Kollision der Querschweißnaht mit der Spitze der Kanüle wird damit ausgeschlossen. Durch anschließendes weiteres Hineindrücken des Schlauchstückes wird die Kanüle in das Innere des Schlauchstückes gedrückt. Ist das Schlauchstück weit genug in das Innere des zylindrischen Körpers hineingeschoben, läßt sich der verbleibende Bereich des Schlauchstückes außerhalb des zylindrischen Körpers zusammendrücken, so daß die darin enthaltene Flüssigkeitsprobe über die Kanüle in ein geeignetes Behältnis zu Testzwecken überführt werden kann.

Bei einer durch die DE 197 34 332 C1 bekanntgewordenen Vorrichtung zur Entnahme einer Flüssigkeitsprobe aus einem Schlauchstück ragt die Kanüle mit ihrem der Spitze entgegengesetzten Ende über das an den zylindrischen Körper angeformte Anschlußstück hinaus. Somit kann auch der über das Anschlußstück hinausragende Teil der Kanüle genutzt werden, um beispielsweise aus dem in die Vorrichtung eingesetzten Schlauchstück bzw. aus einem seiner Schlauchsegmente einen Teil der darin enthaltenen Blutprobe über das freie Ende der Kanüle, welches über das Anschlußstück hinausragt, zu Testzwecken etwa in ein Reagenzglas, ein beliebiges anderes Laborgefäß oder eine Blutproben-Testkarte zu überführen. Dies ist insbesondere bei sogenannten Kreuzproben von Blutproben notwendig. So kann z.B. der über das Anschlußstück hinausragende Teil der Kanüle in eine ein Reagens enthaltende Kammer einer Blutproben-Testkarte, die üblicherweise durch eine durchstechbare Folie verschlossen ist, eingeführt werden, indem das freie Ende der Kanüle die Verschlußfolie durchsticht. Manuell wird dann das Schlauchstück bzw. eines seiner Segmente leicht zusammengedrückt, bis ein oder mehrere Tropfen der darin befindlichen Flüssigkeit in die Reagenzkammer fließt, um daraufhin die Flüssigkeit zu testen.

Alle diese bekannten Vorrichtungen erlauben nur die Möglichkeit der Entnahme einer großen Menge der Flüssigkeit bzw. Flüssigkeitsprobe aus einem Schlauch oder Schlauchabschnitt. Eine Entnahme nur eines sehr kleinen Teils der Gesamtmenge zu Testzwecken ist entweder überhaupt nicht möglich oder äußerst schwierig.

Der Erfindung liegt daher die Aufgabe zugrunde, eine Vorrichtung zur Entnahme einer Flüssigkeitsprobe aus einem Schlauchstück der eingangs genannten Art zu schaffen, welche auch die Entnahme einer nur sehr kleinen Teilmenge der Flüssigkeitsprobe aus dem Schlauchstück zur weiteren Verwendung ermöglicht, wobei die Vorrichtung kostengünstig herstellbar und leicht handhabbar sein soll.

Diese Aufgabe wird erfindungsgemäß dadurch gelöst, daß am freien Ende des Anschlußstückes ein stirnseitig offener Behälter-Hohlraum ausgebildet ist. Dieser z.B. durch einen einstückig an das Stirnende des Anschlußstückes angespritzten Ringwand-Fortsatz erreichte Hohlraum läßt sich von vornherein in seinen Abmessungen und damit seinem Aufnahmevolumen so festlegen, daß eine zu Testzwekken gezielt kleine Probenmenge darin gesammelt und dann entnommen werden kann. Die Hohlraum- bzw. Behälterringwand kann dabei in einer Ebene mit dem stumpfen Ende der Kanüle enden, sie kann geringfügig ober- oder unterhalb der Ebene des stumpfen Kanülenendes enden oder das Kanülenende braucht überhaupt nicht in den Hohlraum einzutauchen, d.h. es kann im wesentlichen bündig mit der Stirnwand des Anschlußstückes abschließend angeordnet werden. Es wird in jedem Fall sichergestellt, daß nur eine kleine Teilmenge der Flüssigkeit aus dem Kanülenende heraustritt, wobei diese bei in den Hohlraum hineinragendem oder diesen überragendem Kanülenende an der Mantelfläche der Kanüle zum Hohlraumgrund nach unten abläuft, während bei bündig mit dem Hohlraumgrund bzw. der Stirnwand des Anschlußstückes abschließendem Kanülenende die Flüssigkeit direkt in den kleinen Hohlraum hineingedrückt wird. Abgesehen von der Variation der Länge der den Hohlraum umgebenden Ring- bzw. Behälterwand läßt sich die aufzunehmende Teilmenge einer Flüssigkeitsprobe weiterhin dadurch auf ein bestimmtes Maß festlegen, daß die den Hohlraum begrenzende Ringwand einen Außendurchmesser aufweist, der kleiner ist als der des Anschlußstückes des zylindrischen Körpers. Ein weiteres Bemessungskriterium ist die Dicke der Ringwand.

Beim Gebrauch dieser insbesondere dadurch besonders kostengünstig herstellbaren Vorrichtung, wenn sie mit Ausnahme der Kanüle vollständig als einstückiges Kunststoffspritzgußteil ausgebildet ist, wird das Schlauchstück senkrecht entlang der Längsmittelachse des zylindrischen Körpers oder in schräger Weise seitlich des zylindrischen Körpers eingeführt, indem der Schlauchabschnitt in die zur Spitze der Kanüle fluchtende Ausbuchtung eingelegt wird, so daß die Querschweißnaht ins Innere des zylindrischen Körpers ragt. Da die Ausbuchtung im wesentlichen zur äußersten Spitze der Kanüle fluchtet, gleitet die Querschweißnaht am vorderen Ende des Schlauchstückes ohne Behinderung über die Kanülenspitze hinweg. Damit hintergreift die Querschweißnaht des Schlauchstückes gewissermaßen die Spitze der Kanüle, wodurch diese in jedem Fall hinter der Querschweißnaht in das die Flüssigkeitsprobe enthaltende Segment des Schlauchstückes einsticht. Eine Kollision der Querschweißnaht mit der Spitze der Kanüle wird daher ausgeschlossen. Durch anschließendes weiteres Hineindrücken des Schlauchstückes wird die Kanüle in das Innere des Schlauchstückes gedrückt. Ist das Schlauchstück weit genug in das Innere des zylindrischen Körpers hineingeschoben, wird der verbleibende Bereich des Schlauchstückes außerhalb des zylindrischen Körpers zusammengedrückt, so daß die darin enthaltene Flüssigkeitsprobe über die Kanüle in ein geeignetes Behältnis zu Testzwecken überführt werden kann.

Soll weiterhin nur eine geringe Teilmenge der im Schlauchabschnitt enthaltenen Flüssigkeitsprobe zu beispielsweise Testzwecken entnommen werden, wird die Vorrichtung zusammen mit dem darin aufgenommenen Schlauchstück einfach herumgeschwenkt, so daß der durch die Behälter- bzw. Ringwand gebildete Hohlraum nach oben weist. Dann wird das Schlauchsegment leicht gedrückt, bis eine kleine Teilmenge der Flüssigkeitsprobe aus dem Kanülenende heraustritt und in den Hohlraum läuft. Durch Loslassen des zusammengedrückten Schlauchstückes kann überschüssige Flüssigkeit gegebenenfalls aus dem Reservoir bzw. Hohlraum abgesaugt werden. Mit einer geeigneten Pipette kann stets die gewünschte Teilmenge der Flüssigkeitsprobe aus dem Flüssigkeitsreservoir entnommen werden.

Die Verwendung der Entnahmevorrichtung ist nicht auf ein Schlauchstück mit einer Flüssigkeitsprobe beschränkt. Vielmehr kann das Schlauchstück auch als aus mehreren Kammern zur Aufnahme von Flüssigkeitsproben bestehender Schlauch ausgebildet sein. Diese Kammern sind jeweils durch eine Querschweißnaht voneinander getrennt. In diesem Fall erfolgt eine Trennung der die Flüssigkeitsproben enthaltenden Kammern entweder durch einen Scherenschnitt oder durch Brechen einer in der Mitte der Querschweißnaht quer zur Längsrichtung des Schlauchstükkes verlaufenden Schwächungslinie. Bei einer solchen Ausbildung ist das Schlauchstück zur Aufbewahrung mehrerer Flüssigkeitsproben geeignet.

Weitere Merkmale und Vorteile der Erfindung ergeben sich aus dem Anspruch und der nachfolgenden Beschreibung, in der in den Zeichnungen dargestellte Ausführungsbeispiele des Gegenstandes der Erfindung näher erläutert sind. Es zeigen:
- Fig. 1: eine im Teil-Längsschnitt dargestellte Gesamtansicht einer Vorrichtung zur Entnahme einer Flüssigkeitsprobe aus einem Schlauchstück mit endseitig ausgebildetem Behälter-Hohlraum, in den das stumpfe Ende einer Kanüle unterhalb der Behälterrandes endet;
- Fig. 2: eine Ansicht gemäß Fig. 1 mit demgegenüber im wesentlichen bündig mit dem Behälter- bzw. Hohlraumgrund abschließenden Kanülenende;
- Fig. 3: eine Ansicht gemäß Fig. 1 mit demgegenüber in einer Ebene mit dem oberen Rand der Behälterwandung abschließenden Kanülenende;
- Fig. 4: eine Ansicht gemäß Fig. 1 mit demgegenüber aus dem Hohlraum nach außen vorragendem Kanülenende;
- Fig. 5: die Vorrichtung nach Fig. 1 in der Draufsicht;
- Fig. 6: einen Schnitt entlang der Linie VI-VI von Fig. 5;
- Fig. 7: einen Schnitt entlang der Linie VII-VII von Fig. 5;
- Fig. 8: in der Draufsicht eine andere Ausführung einer Entnahmevorrichtug einer Flüssigkeitsprobe aus einem Schlauchstück;
- Fig. 9: einen Schnitt entlang der Linie VIIII-VIIII von Fig. 8; und
- Fig. 10: einen Schnitt entlang der Linie X-X von Fig. 8.

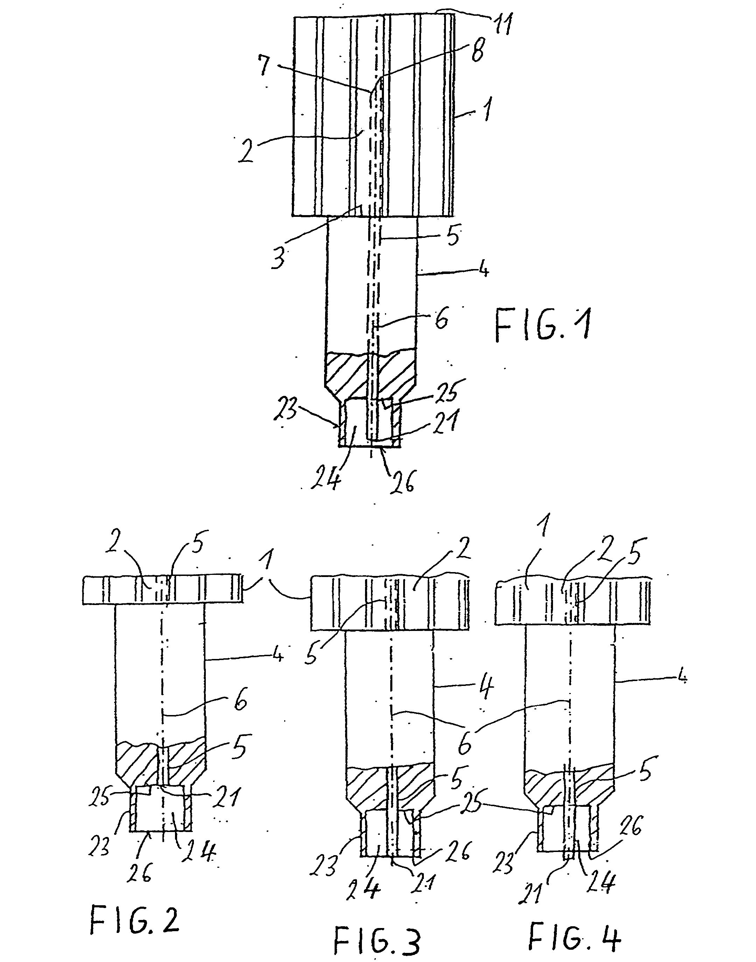

Eine nachfolgend am Beispiel der Kanülen-Anordnung gemäß Fig. 1 beschriebene Vorrichtung zur Entnahme einer Flüssigkeitsprobe besteht im wesentlichen aus einem zylindrischen Körper 1, der in seinem Inneren eine Sacklochöffnung 2 aufweist. Unterhalb des Bodenbereiches 3 der Sacklochöffnung 2 ist am zylindrischen Körper 1 ein Anschlußstück 4 angeformt. Durch dieses Anschlußstück 4 erstreckt sich eine Kanüle 5, die in die Sacklochöffnung 2 im zylindrischen Körper 1 mündet. Die Kanüle 5 verläuft in einer Durchgangsbohrung in der Körperlängsachse 6 des zylindrischen Körpers 1 und weist an ihrem vorderen Ende einen Facettenschliff 7 auf, so daß die äußerste, angeschärfte Spitze 8 der Kanüle 8 leicht seitlich beabstandet zur Körperlängsachse 6 des zylindrischen Körpers 1 ausgerichtet ist.

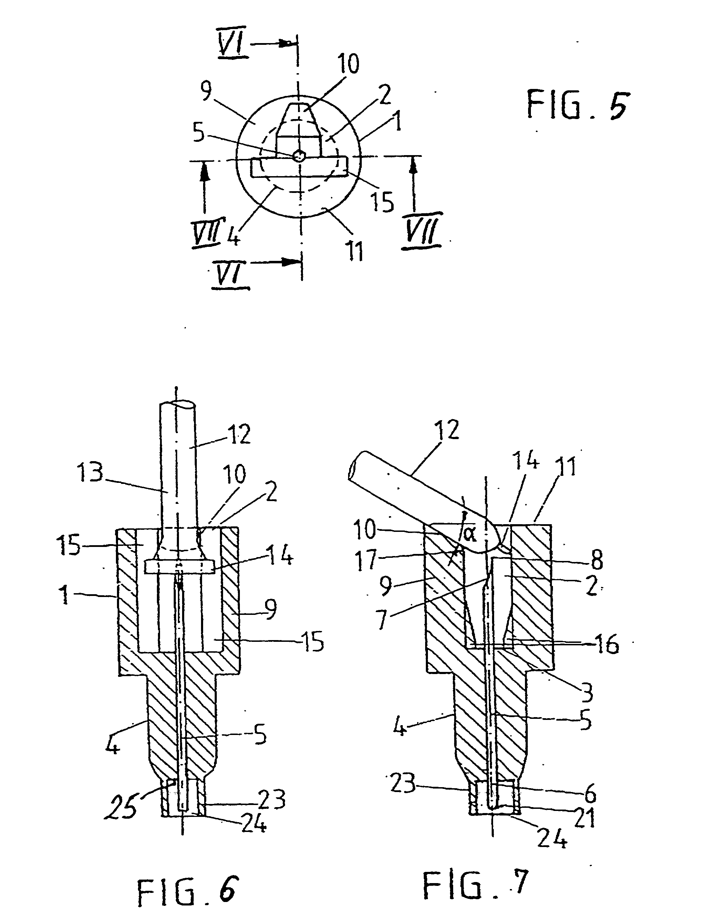

Bei der Ausführungsform der Vorrichtung nach den Fig. 1 bis 7 ist in die innere Umfangswand 9 eine Ausbuchtung 10 eingeformt (vgl. die Fig. 5 bis 7), die etwa in einem Winkel α von 20° bis 60° zur oben offenen Stirnseite 11 des zylindrischen Körpers 1 geneigt ist. Die Fläche der Ausbuchtung 10 bildet hiermit eine gerade Linie mit der äußeren Spitze 8 der Kanüle 5.

Diese Vorrichtung dient zum Öffnen eines Schlauchstückes 12, welches mit einem Schlauchsegment 13 versehen ist, dessen vorderes Ende durch eine Querschweißnaht 14 verschlossen ist (vgl. die Fig. 6), die dadurch zwangsläufig eine größere Breite aufweist als der Durchmesser des Schlauchstückes 12. Dazu korrespondierend weist die Sacklochöffnung 2 des zylindrischen Körpers 1 einen Querschnittsbereich 15 auf, der im wesentlichen der Form der Querschweißnaht 14 des Schlauchstückes 12 entspricht und in parallelem Abstand zur Körperlängsachse 6 des zylindrischen Körpers 1 verläuft. Zweckmäßigerweise ist dieser Querschnittsbereich 15 geringfügig größer, um die Querschweißnaht 14 des Schlauchstückes 12 mit etwas Spiel aufzunehmen.

Gemäß Fig. 7 wird das Schlauchstück 12 derart in die Ausbuchtung 10 der Umfangswand 9 eingelegt, daß die Querschweißnaht 14 mit der der Ausbuchtung 10 gegenüberliegenden Seite der Sacklochöffnung 2 in Anlage kommt. Damit befindet sich die Spitze 8 der Kanüle 5 in Einführrichtung des Schlauchstückes 12 vor der Querschweißnaht 14. Beim weiteren Einschieben des Schlauchstückes 12 durchsticht die Spitze 8 der Kanüle zwangsläufig das Schlauchstück 12 unmittelbar neben dessen Querschweißnaht 14. Beim weiteren Einführen des Schlauchstückes 12 wird dann dessen vorderes Ende in Richtung der Körperlängsachse 6 des zylindrischen Körpers 1 weitergeführt, bis das vordere Ende des Schlauchstückes 12 durch eine an der Innenwand der Sacklochöffnung 2 ausgebildete konische Verjüngung 16 festgehalten wird. Damit kann die im Schlauchsegment 13 des Schlauchstückes 12 befindliche Flüssigkeitsprobe ungehindert über die Kanüle 5 entnommen werden. Weiterhin ist das zur Spitze 8 der Kanüle 5 gerichtete Ende der Ausbuchtung 10 mit einer Abrundung 17 versehen, damit das Einführen des Schlauchstückes 12 weiter erleichtert wird.

Am freien Ende bzw. der Stirnwand 25 des Anschlußstückes 4 des Grundkörpers 1 ist ein durch eine Behälter- bzw. Ringwand 23 begrenzter Hohlraum 24 ausgebildet. Dieser dient als Reservoir für eine daraus beispielsweise zu Testzwecken zu entnehmende geringe Flüssigkeitsmenge, die dort über die Kanüle 5 hin gelangt und sich darin sammeln kann. Zum Überleiten der Flüssigkeit aus dem Schlauchsegment 13 in den Hohlraum 24 sind die in den Fig. 1 bis 4 dargestellten verschiedenen Varianten der Zuordnung des stumpfen Endes 21 der Kanüle 5 in Bezug auf die Ringwand 23 möglich. Nach Fig. 1 ragt die Kanüle 5 über das gleichzeitig den Hohlraum-Grund bildende Stirnende 25 des Anschlußstückes hinaus und endet unterhalb der Oberkante 26 der Ringwand 23, während bei der Ausführung nach den Fig. 3 bzw. 4 das stumpfe Ende 21 der Kanüle 5 in einer Ebene mit der Oberkante 26 liegt bzw. gemäß Fig. 4 über diese hinausragt und nach Fig. 2 bündig mit dem Hohlraum-Grund bzw. der Stirnwand 25 des Anschlußstückes 5 abschließt.

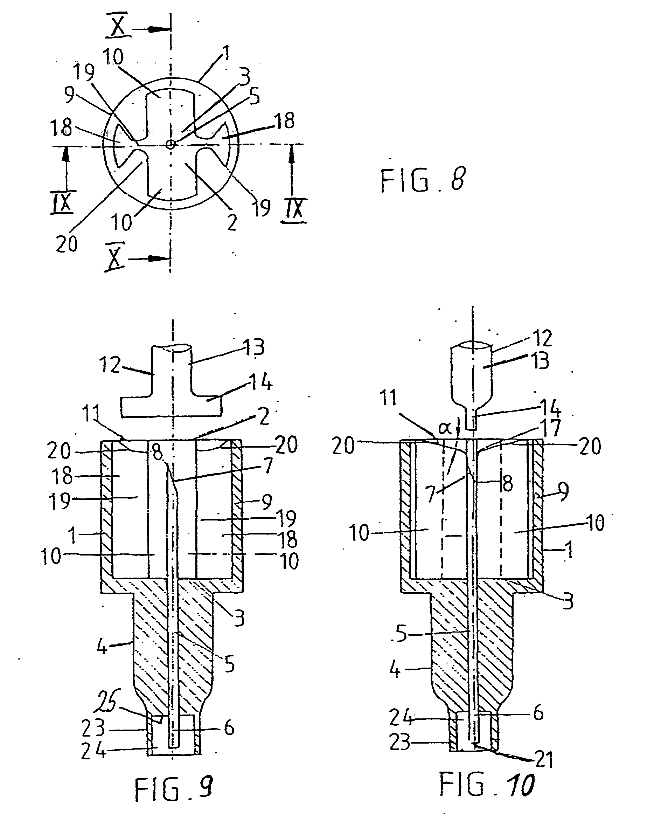

Bei der Ausführungsform der Vorrichtung zur Entnahme einer Flüssigkeitsprobe nach den Fig. 8 bis 10 weist die Sacklochöffnung 2 des zylindrischen Körpers 1 zwei sich diametral gegenüberliegende Ausbuchtungen 10 auf, die senkrecht nach unten bis zum Bodenbereich 3 der Sacklochöffnung 2 verlaufen und zum Aufnehmen des vorderen Teils des Schlauchstückes 12 vorgesehen sind. Ein an seinen äußeren Enden mit jeweils einer Verbreiterung 18 versehener Spaltbereich 19 ist quer zu den Ausbuchtungen 10 verlaufend angeordnet, so daß die Ausbuchtungen 10 und der Spaltbereich 19 im Querschnitt eine Kreuzform bilden. Der Spaltbereich 19, der ebenfalls senkrecht bis zum Bodenbereich 3 der Sacklochöffnung 2 im zylindrischen Körper 1 fluchtet, dient der Aufnahme der Querschweißnaht 14 des Schlauchstückes 12. Die an den äußeren Enden des Spaltbereiches 19 ausgebildeten kreissektorförmigen Verbreiterungen 18 sind vorgesehen, um gegebenenfalls einen verdickte Enden aufweisenden Bereich der Querschweißnaht 14 des Schlauchstückes 12 aufnehmen zu können. Die auf der Stirnseite 11 des zulindrischen Körpers 1 verbleibenden Flächenabschnitte 20, die an einerseits die Ausbuchtungen 10 und andererseits den Spaltbereich 19 angrenzen, sind leicht zur Längsachse des Spaltbereiches 10 geneigt, so daß beim Einschieben des Schlauchstückes 12 die zugehörige Querschweißnaht 14 gegebenenfalls an diesen Flächenabschnitten 20 entlanggleiten kann, um das Einführen der Querschweißnaht 14 in den Spaltbereich 19 zu erleichtern. Hierbei sind auch die vorderen, zum Spaltbereich 19 gerichteten Enden der Flächenabschnitte 20 mit einer Abrundung 17 versehen.

Bei allen Ausführungen schließt sich an das freie Ende des Anschlußstückes 4 die mit relativ geringer Wanddicke ausgebildete, den Hohlraum 24 begrenzende Ringwand 23 an. Wird die Vorrichtung um 180° so gedreht, daß der Hohlraum 24 nach oben weist, kann durch Drücken des Schlauchsegmentes 13 des in die Vorrichtung eingesetzten Schlauchstückes 12 die durch die Kanüle 5 hindurchgepreßte Flüssigkeit an dem stumpfen Ende 21 der Kanüle 5 austreten und in den Hohlraum fließen. Mit einer geeigneten, nicht dargestellten Pipette kann nun die sich im Hohlraum angesammelte kleine Flüssigkeitsmenge zu Testzwecken entnommen werden.

## Patentansprüche

1. Vorrichtung zur Entnahme einer Flüssigkeitsprobe aus einem Schlauchstück (12), das eine verbreiterte Querschweißnaht (14) aufweist, mit einem zylindrischen Körper (1) mit einer Sacklochöffnung (2), einer an deren Umfangswand (9) ausgebildeten, der Querschnittsform der Querschweißnaht (14) des Schlauchstückes (12) entsprechenden Ausbuchtung (10) zum Einsetzen der Querschweißnaht (14) des Schlauchstückes (12) und einer in der Längsachse (6) angeordneten Kanüle (5), die in einer konzentrisch zur Längsachse (6) verlaufenden Durchgangsbohrung eines an den Körper (1) angeformten Anschlußstückes (4) gehalten ist,
**dadurch gekennzeichnet,**
**daß** am freien Ende des Anschlußstückes (4) ein stirnseitig offener Behälter-Hohlraum (24) ausgebildet ist.

## Claims

1. A device for drawing a liquid sample from a flexible tube portion (12) comprising a widened transverse weld seam (14), with a cylindrical body (1) having a blind hole aperture (2), a bight portion (10) formed on the perimeter wall (9) thereof so as to conform to the cross-sectional shape of said transverse weld seam (14) of said flexible tube portion (12) for receiving said transverse weld seam (14) of said flexible tube portion (12) and a canula (5), which is disposed in the longitudinal axis (6) and is retained in a through hole of a fitting (4) integrally formed with the body (1), said through hole being oriented concentrically with the longitudinal axis (6),
**characterized in that**
a hollow enclosure (24) of a container, which is open on its front side, is formed at the free end of the fitting (4).

## Revendications

1. Dispositif pour prélever un échantillon de liquide d'un morceau de tuyau souple (12) comportant un cordon de soudure transversal (14) élargi, avec un corps cylindrique (1) dans lequel débouche un trou borgne (2), avec une échancrure (10) correspondant à la forme de la section du cordon de soudure transversal (14) du morceau de tuyau souple (12) ménagée dans la paroi périphérique (9) dudit trou et destinée à recevoir le cordon de soudure transversal (14) du morceau de tuyau souple (12) et avec une canule (5) disposée dans l'axe longitudinal (6) et retenue dans un trou traversant concentrique à l'axe longitudinal (6) ménagé dans un raccord (4) issu de moulage avec le corps (1),
**caractérisé en ce**
**qu'**est formée, à l'extrémité libre du raccord (4), une cavité (24) d'un récipient ouvert sur sa face frontale.
